# EUROPEAN PATENT APPLICATION

(11) **EP 1 974 739 A1**
(43) Date of publication of application: **01.10.2008**
(21) Application number: 07005784.9
(22) Date of filing: 21.03.2007
(51) Int. Cl.: A61K 38/06, A61K 31/192, A61K 31/195, A61K 31/405, A61K 31/4152, A61K 31/4704, A61K 31/519, A61K 31/5575, A61K 31/575, A61P 29/00, A61P 11/00

(54) **Methods and compounds for modulating inflammatory processes**

(71) Applicant: Deutsches Krebsforschungszentrum, Stiftung des öffentlichen Rechts, 69120 Heidelberg (DE)
(72) Inventor: Rius Montraveta, Maria, 69121, Heidelberg (DE); Hummel-Gisenbeiß, 69123, Heidelberg (DE); Keppler, Dietrich, 69221, Dossenheim (DE)
(74) Representative: Schüssler, Andrea

(57) **Abstract**

Provided is a method of treating a patient having an inflammatory disease by using a compound which inhibits the ABCC4-mediated release of leukotrienes, a medicament containing such compound and methods for screening such compounds.

## Description

The present invention refers to a method for modulating inflammatory processes by selectively inhibiting the multidrug resistance protein ABCC4.

### INTRODUCTION

Leukotrienes (LTs) are biologically potent proinflammatory lipid mediators mainly derived from arachidonate (for reviews see 1-3). The biosynthesis of LTB₄ and of LTC₄ is followed by a distinct export step recognized originally in human leukocytes (4-6), murine mastocytoma cells (7), and plasma membrane vesicles from normal and transport-deficient rat hepatocytes (8,9). Efflux transport of LTB₄ and LTC₄ is an essential step in cells containing all enzymes of LT biosynthesis (for review see 2) and also in transcellular LT biosynthesis (reviewed by 3): Thus, LTA₄ taken up from other cells was actively converted to LTC₄ in platelets and effluxed by an energy-dependent export pump (10,11). Moreover, erythrocytes converted exogenous LTA₄ to LTB₄ which was subsequently exported into the extracellular space (12).

The members of the multidrug resistance protein subfamily, including ABCC1-6 and ABCC10-12, have a rather broad substrate specificity and mediate the ATP-dependent efflux of organic anions, including glutathione conjugates such as LTC₄, across the plasma membrane into the extracellular space (for reviews see 17-21). In the case of ABCC1 (22,23) and ABCC4 (MRP4; 24,25), it was recognized however, that the presence or the co-transport of reduced glutathione (GSH) may change the substrate specificity and allow the efflux of substances that are not transported in the absence of GSH. GSH occurs in living cells at millimolar concentrations (26), and modulation of the substrate specificity of ABCC transporters by GSH represents a physiological condition. For example, we showed that ABCC4 transports most bile acids only in the presence of GSH (24,25).

The multidrug resistance protein 1 (MRP1; 13), now termed ATP-binding cassette transporter, subfamily C, member 1 (ABCC1) was the first ATP-dependent export pump identified for LTC₄ (14,15). The link between ABCC1 and LTC₄ as its substrate was established primarily by photoaffinity labeling studies using LTC₄ with its conjugated triene structure as a high-affinity ligand and the LTD₄ receptor antagonist MK571 as a competitive inhibitor of photoaffinity labeling and transport (14-16). However, ABCC1 does not transport LTB₄. Thus, the molecular identity of the biologically important efflux transporter for LTB₄ has been unknown.

Based on the knowledge from the prior art, the inventors studied the ATP-dependent transport of LTB₄ and LTC₄ by ABCC4 in the presence or absence of GSH. ABCC4 is widely distributed in cells and tissues, including prostate (27,28), urogenital tissues (29), kidney proximal tubules (30), choroid plexus epithelial cells (31), astrocytes and capillary endothelial cells of the brain (32), platelets (33), erythrocytes (34), hepatocytes (24), and many cancer cells lines (35,36). The present invention describes the role of ABCC4 in the ATP-dependent efflux transport of LTB₄ and LTC₄ from cells.

### DETAILED DESCRIPTION

WO 2005/044244 describes the use of certain ABCC4 inhibitors for the treatment of patients suffering from cardiovascular diseases. However, the application does not provide any guidance whether the disclosed inhibitors are capable of modulating inflammatory processes. Furthermore, the inhibitors disclosed in the WO 2005/044244 are selected for inhibiting the transport of nucleotides such as ADP via ABCC4, and, consequently, do not address the treatment of inflammatory diseases resulting from an increased leukotriene release.

Furthermore, the WO 2006/134022 discloses the use of certain ABC inhibitors for the treatment of patients suffering from respiratory diseases. However, WO 2006/134022 does not disclose inhibitors which selectively block the transport of leukotrienes out of cells.

Thus, the problem underlying the present invention is to provide means for selectively inhibit the efflux of mediators of inflammatory diseases ABCC4 in order to prevent those mediators of inflammatory diseases to reach their site of action and cause inflammatory processes.

The problem is solved by the present invention, which provides method of treating a patient having an inflammatory disease, wherein the method comprises using a therapeutically effective amount of a compound which inhibits the ABCC4-mediated release of leukotrienes.

For the purpose of convenience, the term "compound which inhibits the ABCC4-mediated release of leukotrienes" is hereinafter referred to as "leukotriene release inhibitor".

As used herein, the term "treating" or "treatment" as used in relation to the treatment of inflammatory diseases is to be understood as embracing both symptomatic and prophylactic modes, that is the immediate treatment, e.g. of acute inflammation (symptomatic treatment) as well as advance treatment to prevent, ameliorate or restrict long term symptomatology (prophylactic treatment). The term "treatment" as used in the present specification and claims in relation to such diseases is to be interpreted accordingly as including both symptomatic and prophylactic treatment, e.g., in the case of asthma, symptomatic treatment to ameliorate acute inflammatory events and prophylactic treatment to inhibit ongoing inflammatory status and to ameliorate future bronchial exacerbation associated therewith.

As used herein, the term "inflammatory disease" refer to diseases which are a result of an individual's reaction of connecting tissue and blood vessels to an external or internal inflammation stimulus, with the purpose to eliminate or inactivate said stimulus. Triggering effectors include mechanical stimuli, or other physical factors, e.g. ionizing radiation, UV light, heat, coldness; chemical substances, e.g. bases, acids, heavy metals, bacterial toxins, allergens, and immune complexes, as well as pathogens, e.g. microorganisms and viruses, worms and insects; and pathogenic metabolism products, malfunctioning enzymes, malign tumors.

As used herein, "therapeutically effective amount" of one of the leukotriene release inhibitor means a sufficient amount of said compound to treat a particular disease, at a reasonable benefit/risk ratio, In general, the term "therapeutically effective amount" shall refer to an amount of said compound which is physiologically significant and improves an individual's health. An agent, i.e. said compound, is physiologically significant if its presence results in a change in the physiology of the recipient human. For example, in the treatment of a pathological condition, administration of said compound which relieves or arrests further progress of the condition would be considered both physiologically significant and therapeutically effective. Said compound may be employed in pure form or, where such forms exist, in pharmaceutically acceptable salt, ester or prodrug forms.

As used herein, the term "pharmaceutically acceptable salt" refers to those salts which are, within the scope of sound medical judgement, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge, et al. describe pharmaceutically acceptable salts in detail in J. Pharmaceutical Sciences, 66; 1-19 (1977), which is incorporated herein by reference. The salts can be prepared in situ during the final isolation and purification of the leukotriene release inhibitors, or separately by reacting the free base function with a suitable organic acid. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, berate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydmxy-ethanesulfonate, lactobionate, lactate, laurate, lauryl sulfate, malate, maleate, malonate, methanesulfonate, 2-naphthalenesulfonate, nicotinate; nitrate, oleate, oxalate, palmitate, pamoate, pectinate, persulfate. 3-phenylpropionate, phosphate, picrate, pivalate, propionate, stearate, succinate, sulfate, tartrate, thiocyanate, p-tolueneswfonate, undecanoate, vale rate salts, and the like. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

As described supra, the leukotriene release inhibitors are useful in the treatment of inflammatory diseases, particularly inflammatory diseases which may arise due to an increased leukotriene release in response to an external or internal inflammation stimulus. Such inflammatory diseases can be subdivided into chronic inflammatory diseases, acute inflammatory diseases, allergic inflammatory disorders, graft-versus-host rejection diseases, and autoimmune disorders, all of which are encompassed within the present invention. Among these subgroups, inflammatory diseases of the respiratory tract, inflammatory skin diseases, allergic inflammatory disorders, inflammatory diseases of the gastrointestinal tract and inflammatory heart diseases can occur.

Accordingly, leukotriene release inhibitors are useful for the treatment of diseases or conditions responsive to or requiring anti-inflammatory, immunosuppressive or related therapy, including topical administration for the treatment of such diseases or conditions of the eye, nasal passages, buccal cavity, skin, heart, colon or, especially, airways or lung. In particular leukotriene release inhibitors permit topical anti-inflammatory, immunosuppressive or related therapy with the concomitant avoidance or reduction of undesirable systemic side effects, for example renal toxicity or general systemic immunosuppression.

Leukotriene release inhibitors are particularly useful for the treatment of diseases and conditions of the airways or lung, i.e diseases of the respiratory tract, in particular inflammatory or obstructive airways disease. They are especially useful for the treatment of diseases or conditions of the airways or lung associated with or characterized by inflammatory cell infiltration or other inflammatory event accompanied by the accumulation of inflammatory cells, e.g. eosinophils and/or neutrophils.

In this respect, Leukotriene release inhibitors are useful in the treatment of asthma of whatever type of genesis including both intrinsic and, especially, extrinsic asthma. They are useful for the treatment of atopic and non-atopic asthma, including allergic asthma, bronchitic asthma, exercise-induced asthma, occupational asthma, asthma induced following bacterial infection and other non-allergic asthmas. Leukotriene release inhibitors are also useful for the treatment of bronchitis or for the treatment of chronic or acute airways obstruction associated therewith. Leukotriene release inhibitors may be used for the treatment of bronchitis of whatever type or genesis, including, for example, acute bronchitis, arachidic bronchitis, catarrhal bronchitis, chronic bronchitis, croupous bronchitis, phthinoid bronchitis and so forth. Leukotriene release inhibitors are in addition useful for the treatment of pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, berylliosis chalicosis, ptilosis, siderosis, silicosis, tabacosis and, in particular, byssinosis. Leukotriene release inhibitors may also be used to treat any disease or condition of the airways or lung requiring immunosuppressive therapy, e.g., for the treatment of autoimmune diseases of, or as they affect, the lungs (for example, for the treatment of sarcoidosis, alveolitis or chronic hypersensitivity pneumonitis) or for the maintainance of allogenic lung transplant, e.g., following lung or heart lung transplantation.

For the above purposes, some leukotriene release inhibitors preferably will be administered topically within the airways, e.g. by the pulmonary route, by inhalation. While having potent efficacy when administered topically, leukotriene release inhibitors are devoid of, or exhibit relatively reduced, systemic activity, e.g. following oral administration. Leukotriene release inhibitors thus provide a means for the treatment of diseases and conditions of the airways or lung with the avoidance of unwanted systemic side effect, e.g., consequent to inadvertent swallowing of drug substance during inhalation therapy. (It is estimated that during the course of maneuvers required to effect administration by inhalation, up to 90% or more of total drug substance administered will inadvertently be swallowed rather than inhaled). By the provision of leukotriene release inhibitors which are topically active, e.g. effective when inhaled but systemically inactive, the present invention makes leukotriene release inhibitor therapy available to subjects for whom such therapy might otherwise be excluded, e.g., due to the risk of systemic, in particular immunosuppressive, side effects.

Leukotriene release inhibitors are also useful for the treatment of other diseases or conditions, in particular diseases, or conditions having an autoimmune or inflammatory component and for which topical therapy may be practiced, for example, treatment of diseases and conditions of the eye such as conjunctivitis, keratoconjunctivitis sicca, and vernal conjunctivitis and maintenance of corneal transplant, disease affecting the nose including allergic rhinitis, diseases and conditions of the skin including psoriasis, atopic dermatitis, pemphigus and contact dermatitis, as well as diseases of the colon, for example Crohn's disease and ulcerative colitis.

As immunosuppressants, leukotriene release inhibitors are useful when administered for the prevention of immune-mediated tissue or organ graft rejection. Examples of transplanted tissues and organs which suffer from these effects are heart, kidney, liver, medulla ossium, skin, cornea, lung, pancreas, intestinum tenue, limb, muscle, nervus, duodenum, small-bowel, pancreatic-islet-cell, and the like; as well as graft-versus-host diseases brought about by medulla ossium transplantation.

The regulation of the immune response by leukotriene release inhibitors would also find utility in the treatment of autoimmune disorders, such as rheumatoid arthritis, systemic lupus erythematosis, hyperimmunoglobulin E, Hashimoto's thyroiditis, multiple sclerosis, progressive systemic sclerosis, myasthenia gravis, type I diabetes, uveitis, allergic encephalomyelitis, glomerulonephritis, and the like; and further infectious diseases caused by pathogenic microorganisms, such as HIV. In the particular cases of HIV-1, HIV-2 and related retroviral strains, inhibition of T-cell mitosis would suppress the replication of the virus, since the virus relies upon the host T-cell's proliferative functions to replicate.

Further uses include the treatment and prophylaxis of inflammatory and hyperproliferative skin diseases and cutaneous maniffestations of immunologically-mediated illnesses, such as psoriasis, atopical dermatitis, contact dermatitis and further eczematous dermatitises, seborrhoeis dermatitis, Lichen planus, Pemphigus, bullous pemphigoid, Epidermolysis bullosa, urticaria, angioedemas, vasculitides, erythemas, cutaneous eosinophilias, Lupus erythematosus, acne and Alopecia areata; various eye diseases (autoimmune and otherwise) such as keratoconjunctivitis, vernal conjunctivitis, keratitis, herpetic keratitis, conical cornea, dystrophia epithelialis corneae, corneal leukoma, ocular pemphigus, Mooren's ulcer, Scleritis, Graves' opthalmopathy, Vogt-Koyanagi-Harada syndrome, sarcoidosis, multiple myeloma, etc.; inflammation of mucosa and blood vessels such as gastric ulcers, vascular damage caused by ischemic diseases and thrombosis. Moreover, hyperproliferative vascular diseases such as intimal smooth muscle cell hyperplasia, restenosis and vascular occlusion, particularly following biologically- or mechanically-mediated vascular injury can be treated or prevented by leukotriene release inhibitors.

Other treatable conditions would include but are not limited to ischemic bowel diseases, inflammatory bowel diseases, necrotizing enterocolitis, intestinal lesions associated with thermal burns and leukotriene B4-mediated diseases; intestinal inflammations/allergies such as Coeliac diseases, proctitis, eosinophilic gastroenteritis, and mastocytosis; food-related allergic diseases which have symptomatic manifestation remote from the gastrointestinal tract (e.g., migraine, rhinitis and eczema); renal diseases such as interstitial nephritis, Goodpasture's syndrome, hemolytic-uremic syndrome and diabetic nephropathy; nervous diseases such as multiple myositis, Guillain-Barre syndrome, Meniere's disease, polyneuritis, multiple neuritis, mononeuritis and radiculopathy; endocrine diseases such as hyperthyroidism and Basedow's disease; hematic diseases such as pure red cell aplasia, aplastic anemia, hypoplastic anemia, idiopathic thrombocytopenic purpura, autoimmune hemolytic anemia, agranulocytosis, pernicious anemia, megaloblastic anemia: and anerythroplasia; bone diseases such as osteoporosis; respiratory diseases such as sarcoidosis, fibroid lung and idiopathic interstitial pneumonia; skin disease such as dermatomyositis, leukoderma vulgaris, ichthyosis vulgaris, photoallergic sensitivity and cutaneous T cell lymphoma; circulatory diseases such as arteriosclerosis, atherosclerosis, aortitis syndrome, polyarteritis nodosa and myocardosis; collagen diseases such as scleroderma, Wegener's granuloma and Sjogren's syndrome; adiposis; eosinophilic fasciitis; periodontal disease such as lesions of gingiva, periodontium, alveolar bone and substantia ossea dentis; nephrotic syndrome such as glomerulonephritis; male pattern aleopecia or alopecia senilis by preventing epilation or providing hair germination and/or promoting hair generation and hair growth; muscular dystrophy; Pyoderma and Sezary's syndrome; Addison's disease; active oxygen-mediated diseases, as for example organ injury such as ischemia-reperfusion injury of organs (such as heart, liver, kidney and digestive tract) which occurs upon preservation, transplantation or ischemic disease (for example, thrombosis and cardiac infraction): intestinal diseases such as endotoxin-shock, pseudomembranous colitis and colitis caused by drug or radiation; renal diseases such as ischemic acute renal insufficiency and chronic renal insufficiency; pulmonary diseases such as toxinosis caused by lung-oxygen or drug (for example, paracort and bleomycins), lung cancer and pulmonary emphysema; ocular diseases such as cataracta, siderosis, retinitis, pigmentosa, senile macular degeneration, vitreal scarring and corneal alkali burn; dermatitis such as erythema multiforme, linear IgA ballous dermatitis and cement dermatitis; and others such as gingivitis, periodontitis, sepsis, pancreatitis, diseases caused by environmental pollution (for example, air pollution), aging, carcinogenis, metastasis of carcinoma and hypobaropathy; disease caused by histamine or leukotriene-C4 release; Behcet's disease such as intestinal-, vasculo- or neuro-Behcet's disease, and also Behcet's which affects the oral cavity, skin, eye, vulva, articulation, epididymis, lung, kidney and so on.

Aqueous liquid compositions of the present invention may be particularly useful for the treatment and prevention of various diseases of the eye such as autoimmune diseases (including, for example, conical cornea, keratitis, dysophia epithelialis corneae, leukoma, Mooren's ulcer, sclevitis and Graves' ophthalmopathy) and rejection of corneal transplantation. In particular, compositions pertaining to the present invention are useful for treating a subject for immune-mediated organ or tissue allograft rejection, a graft-versus-host disease, an autoimmune disease, an obstructive airway disease, a hyperproliferative disease, or an ischemic or inflammatory intestinal or bowel disease.

Accordingly, the present invention further refers to pharmaceutical preparations for the treatment of inflammatory diseases which comprises a leukotriene release inhibitor and, optionally, a pharmaceutically acceptable carrier, and methods for preparing such pharmaceutical compositions. The methods for preparing pharmace tical compositions, i.e. medicaments, are known per se to the skilled artisan.

The specific therapeutically-effective dose level for any particular patient will depend upon a variety of factors including the disorder being treated and the severity of the disorder; activity of the specific leukotriene release inhibitor employed; the specific composition employed; the age, body weight, general health, sex and diet of the patient; the time of administration, route of administration, and rate of excretion of the specific leukotriene release inhibitor employed; the duration of the treatment; drugs used in combination or coincidental with the specific leukotriene release inhibitor employed; and like factors well known in the medical arts. For example, it is well within the skill of the art to start doses of the leukotriene release inhibitor at levels lower than required to achieve the desired therapeutic effect and to gradually increase the dosage until the desired effect is achieved.

As used herein, the term "pharmaceutically acceptable carrier" means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols, such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, phosphate buffer solutions; non-toxic, compatible lubricants such as sodium lauryl sulfate and magnesium stearate; as well as coloring agents, releasing agents, coating agents, sweetening, flavoring and perfuming agents. Preservatives and antioxidants can also be present in the composition, according to the judgment of the formulator.

The compositions may be administered to humans and other animals orally, rectally, parenterally, intracisternally, intravaginally, intraperitoneally, topically (as by powders, ointments, drops or transdermal patch), bucally, or as an oral or nasal spray. The term "parenteral" as used herein refers to modes of administration which include intravenous, intramuscular, intraperitoneal, intrasternal, subcutaneous and intraarticular injection and infusion.

Dosage forms for topical or transdermal administration of leukotriene release inhibitors include ointments, pastes, creams, lotions, gels, plasters, cataplasms, powders, solutions, sprays, inhalants or patches. The active component, i.e. the leukotriene release inhibitor, is admixed under sterile conditions with a pharmaceutically acceptable carrier and any needed preservatives or buffers as may be required. The ointments, pastes, creams and gels may contain, in addition to an active leukotriene release inhibitor of this invention, excipients such as animal and vegetable fats, oils, waxes, paraffins, starch, tragacanth, cellulose derivatives, polyethylene glycols, silicones, bentonites, silicic acid, talc and zinc oxide, or mixtures thereof. Powders and sprays can contain, in addition to leukotriene release inhibitors, excipients such as lactose, talc, silicic acid, aluminum hydroxide, calcium silicates and polyamide powder, or mixtures of these substances. Sprays can additionally contain customary propellants such as chlorofluorohydrocarbons. For nasal administration, leukotriene release inhibitors will suitably be administered in liquid or powdered form from a nasal applicator. Forms suitable for ophthalmic use will include lotions, tinctures, gels, ointment and ophthalmic inserts, again as known in the art. For rectal administration, i.e., for topical therapy of the colon, leukotriene release inhibitors may be administered in suppository or enema form, in particular in solution, e.g., in vegetable oil or like oily system for use as a retention enema.

It is clear that safety may be maximized by delivering the drugs by the inhaled route either in nebuliser form or as dry powder. Clearly the great advantage of the inhaled route, over the systemic route, in the treatment of asthma and other diseases of airflow obstruction and/or of chronic sinusititis, is that patients are exposed to very small quantities of the drug and the leukotriene release inhibitor is delivered directly to the site of action.

Leukotriene release inhibitors therefore are preferably employed in any dosage form appropriate for topical administration to the desired site. Thus, for the treatment of diseases of the airways or lungs, leukotriene release inhibitors may be administered via the pulmonary route/by inhalation from an appropriate dispenser device. For this purpose, leukotriene release inhibitors may be employed in any suitable finely dispersed or finely dispersible form, capable of administration into the airways or lungs, for example in finely divided dry particulate form or in dispersion or solution in any appropriate (i.e., pulmonarily administerable) solid or liquid carrier medium. For administration in dry particulate form, leukotriene release inhibitors may, for example, be employed as such, i.e., in micronised form without any additive materials, in dilution with other appropriate finely divided inert solid carrier or diluent (e.g., glucose, lactose, mannitol, sorbitol, ribose, mannose or xylose), in coated particulate form or in any other appropriate form as known in the art for the pulmonary administration of finely divided solids, Pulmonary administration may be effected using any appropriate system as known in the art for delivering drug substance in dry or liquid form by inhalation, e.g. an atomizer, nebulizer, dry-powder inhaler or like device. Preferably a metered delivery device, i.e., capable of delivering a pre-determined amount of leukotriene release inhibitor at each actuation, will be employed. Such devices are known in the art.

Pharmaceutical compositions of this invention for parenteral injection comprise pharmaceutically-acceptable sterile aqueous or nonaqueous solutions, dispersions, suspensions or emulsions, as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), carboxymethylcellulose and suitable mixtures thereof, vegetable oils (such as olive oil), and injectable organic esters such as ethyl oleate. Proper fluidity may be maintained, for example, by the use of coating materials such as lecithin, by the maintenance of the required particle size in the case of dispersions, and by the use of surfactants.

These compositions may also contain adjuvants such as preservative, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like. Prolonged absorption of the injectable pharmaceutical form may be brought about by the inclusion of agents which delay absorption, such as aluminum monostearate and gelatin.

In some cases, in order to prolong the effect of the drug, it is desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming mieroencapsule matrices of the drug in biodegradable polymers such as polylactide-polyglycolide, pol (orthoesters) and poly(anhydrides). Depending upon the ratio of drug to polymer and the nature of the particular polymer employed, the rate of drug release can be controlled. Depot injectable formulations are also prepared by entrapping the drug in liposomes or microemulsions which are compatible with body tissues. The injectable formulations may be sterilized, for example, by filtration through a bacterial-retaining filter, or by incorporating sterilizing agents in the form of sterile solid compositions which can be dissolved or dispersed in sterile water or other sterile injectable medium just prior to use.

Solid dosage forms for oral administration include capsules, tablets, pills, powders, and granules. In such solid dosage forms, the active leukotriene release inhibitor is mixed with at least one inert, pharmaceutically-acceptable excipient or carrier, such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as tale, calcium stearate, magnesium stearate, solid polyethylene glycols, sodium lauryl sulfate, and mixtures thereof. In the case of capsules, tablets and pills, the dosage form may also comprise buffering agents. Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules may be prepared with coatings and shells such as enteric coatings and other coatings well known in the pharmaceutical formulating art, They may optionally contain opacifying agents and can also be of a composition that they release the active ingredient(s) only, or preferentially, in a certain part of the intestinal tract, optionally, in delayed manner. Examples of embedding compositions which can be used include polymeric substances and waxes.

Liquid dosage forms for oral administration include pharmaceutically-acceptable emulsions, solutions, suspensions, syrups and elixirs. In addition to the active leukotriene release inhibitors, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, com, germ, olive, castor, and sesame oils), glycerol, tetrahydrofurfuryl alcohol, polyethylene glycols and fatty acid esters of sorbitan, and mixtures thereof. Suspensions may contain, in addition to the active leukotriene release inhibitors, suspending agents as, for example, ethoxylated isostearyl alcohols, polyoxyethylene sorbitol and sorbitan esters, microciystalline cellulose, aluminum metahydroxide, bentonite, agar-agar, and tragacanth, and mixtures thereof.

Topical administration includes administration to the skin or mucosa, including surfaces of the lung and eye. Compositions for topical administration, including those for inhalation, may be prepared as a dry powder which may be pressurized or non-pressuhzed. In non-pressurized powder compositions, the active ingredient in finely divided form may be used in admixture with a larger-sized pharmaceutically-acceptable inert carrier comprising particles having a size, for example, of up to 100 micrometers in diameter. Suitable inert carriers include sugars such as lactose. Desirably, at least 95% by weight of the particles of the active ingredient have an effective particle size in the range of 0.01 to 10 micrometers. Alternatively, the composition may be pressurized and contain a compressed gas, such as nitrogen or a liquified gas propellant. The liquified propellant medium and indeed the total composition are preferably such that the active ingredient does not dissolve therein to any substantial extent. The pressurized composition may also contain a surface-active agent, such as a liquid or solid non-ionic surface-active agent or may be a solid anionic surface-active agent. It is preferred to use the solid anionic surface-active agent in the form of a sodium salt.

A further form of topical administration is to the eye, as for the treatment of immune-mediated conditions of the eye such as autoimmune diseases, allergic or inflammatory conditions, and corneal transplants. The leukotriene release inhibitor is delivered in a pharmaceutically acceptable ophthalmic vehicle, such that the leukotriene release inhibitor is maintained in contact with the ocular surface for a sufficient time period to allow the leukotriene release inhibitor to penetrate the corneal and internal regions of the eye, as for example the anterior chamber, posterior chamber, vitreous body, aqueous humor, vitreous humor, cornea, iris/cilary, lens, choroid/retina and sclera. The pharmaceutically acceptable ophthalmic vehicle may, for example, be an ointment, vegetable oil or an encapsulating material.

Leukotriene release inhibitors may also be administered in the form of liposomes. As is known in the art, liposomes are generally derived from phospholipids or other lipid substances Liposomes are formed by mono- or multi-lamellar hydrated liquid crystals that are dispersed in an aqueous medium. Any non-toxic, physiologically acceptable and metabolizable lipid capable of forming Liposomes can be used. The present compositions in liposome form can contain, in addition to a leukotriene release inhibitor, stabilizers, preservatives, excipients, and the like. The preferred lipids are the phospholipids and the phosphatidyl cholines (lecithins), both natural and synthetic. Method to form liposomes are known in the art. See, for example, Prescott, Ed., Methods in Cell Biology, Volume XIV, Academic Press, New York, N.Y. (1976), p. 33 et seq.

In a preferred embodiment, the leukotriene, the release of which shall be inhibited according to the present invention, is leukotriene C4 or B4 (LTB4), most preferably LTB4.

The leukotriene release inhibitor can be any compound that is capable of inhibiting the ABCC4-mediated release of leukotrienes, particularly LTB4 or LTC4, from cells. In a preferred embodiment, the leukotriene release inhibitors are those listed in Table 1.

Most preferably, the leukotriene release inhibitors are indomethacin, sulindac derivatives, such as sulindac sulfide or montelukast,

In another embodiment, the present invention refers to a method of screening for a compound which inhibits the ABCC4-mediated release of leukotrienes the method comprising
(a) contacting cells expressing ABCC4 with the compound under investigation and / or contacting membrane vesicles containing ABCC4 with the compound under investigation, and
(b) measuring the amount of released leukotrienes,
wherein a lower amount of released leukotrienes in the cells / membrane vesicles of (a) as compared to cells or membrane vesicles that are not contacted with said compound is indicative of an inhibitory effect of said compound.

Step (b) of the method of the present invention can be performed by labelling the leukotriene the release of which shall be monitored. In a preferred embodiment, the used leukotriene is radioactively labelled.

Examples of how to perfom the method of invention is described in Example 1 a-g.

As described supra in the present invention, the inflammatory diseases which may be treated by the use of leukotriene release inhibitors, respectively the medicaments containing them, may arise due to an increased release of certain leukotriens such as LTB4 and LTC4.

Therefore, the present invention further refers to a method of inhibiting leukotriene release from cells, the method comprising the step of treating said cells with a leukotriene release inhibitor.

### DESCRIPTION OF THE DRAWINGS

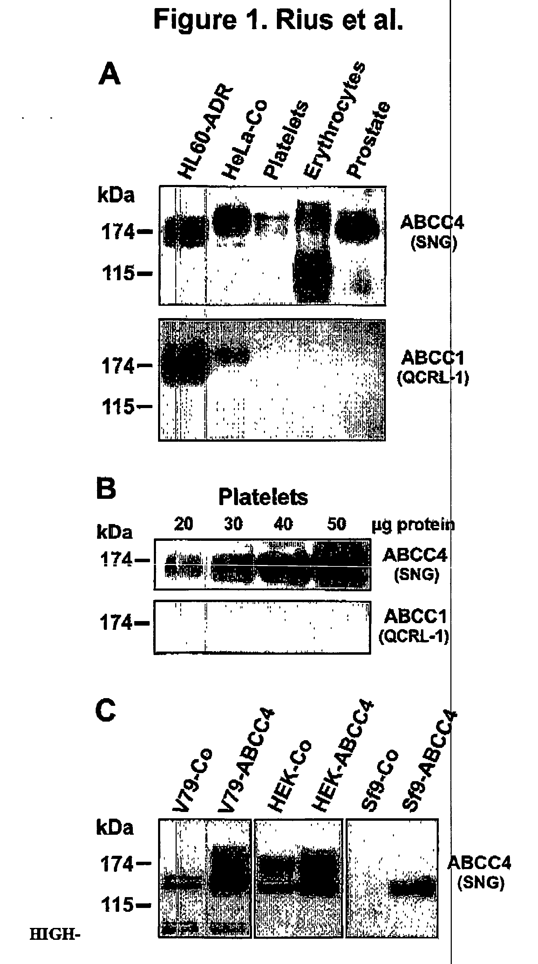

Fig. 1, **Immunodetection of ABCC4 and ABCC1.** A, immunoblot analysis of ABCC4 and ABCC1 in membrane vesicles from HL60-ADR and HeLa control (HeLa-Co) cells, in the plasma membrane fraction from human platelets, in membrane vesicles from human erythrocytes, and tissue homogenate from human prostate (10 µg protein per lane). The blots were immunostained using the polyclonal SNG antiserum against human ABCC4 (24) and the monoclonal QCRL-1 antibody against human ABCC1 (46). Human prostate and HL60-ADR were used as positive controls for ABCC4 (28) and ABCC1, respectively. B, The plasma membrane fraction from human platelets was loaded in a range from 20 µg to 50 µg protein. Increasing amounts of ABCC4 protein were detected by the SNG antiserum but ABCC1 signal intensity remained below detectability using the QCRL-1 antibody and equal exposure times for both blots. C, lmmunoblot analysis of ABCC4 using the SNG antiserum in membrane vesicles (30 µg per lane) from control cells (V79-Co, HEK-Co, and Sf9-Co) and from cells expressing recombinant ABCC4 (V79-ABCC4, HEK-ABCC4, and Sf9-ABCC4).

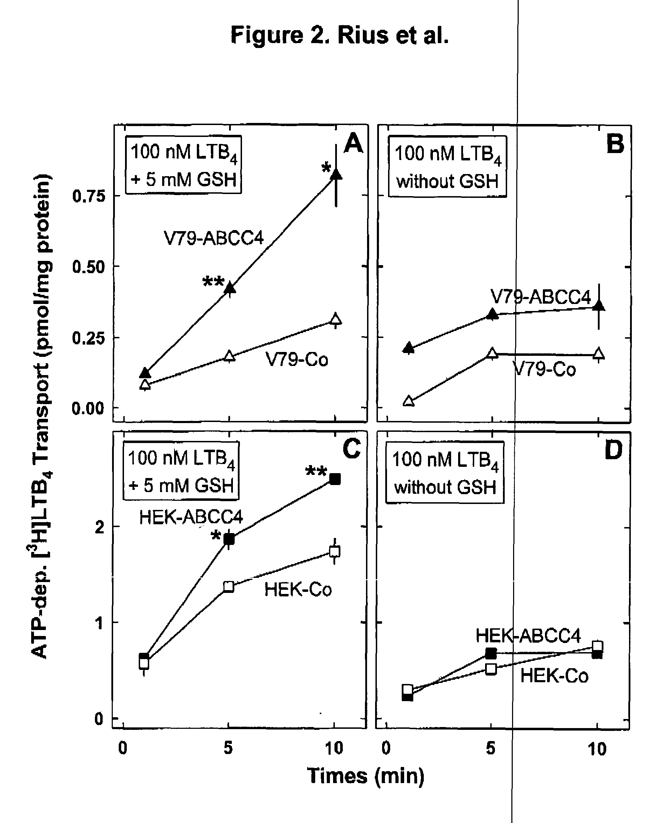

**Fig. 2****. GSH increases transport of LTB₄ into membrane vesicles from ABCC4-transfected (V79-ABCC4 and HEK-ABCC4) and vector-transfected control (V79-Co and HEK-Co) cells.** Membrane vesicles (30 µg of protein) were incubated with 100 nM [³H]LTB₄ in the absence (B and D) or presence (A and C) of 5 mM G SH, and the vesicle-associated radioactivity was determined by centrifugation through Sephadex G-50 columns. The rates of net ATP-dependent transport were calculated by subtracting transport in the presence of 4 mM AMP-PCP as a blank from transport in the presence of 4 mM ATP. Data represent mean values ± S.D. from a triplicate determination reproduced independently at least once. **P*< 0.01 and ***P*<0.001 as compared to vesicles from vector-transfected control cells.

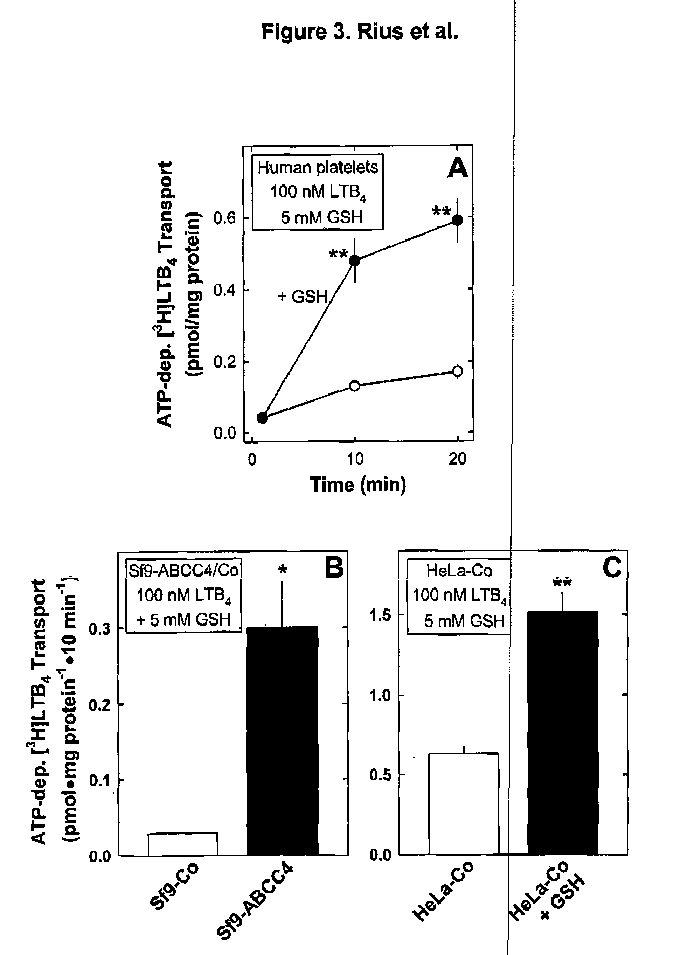

Fig. 3. Effect of GSH on transport of LTB₄ into membrane vesicles from human platelets, from Sf9 cells containing recombinant ABCC4 protein, and from HeLa control cells. A,
   Membrane vesicles (100 µg of protein) from human platelets were incubated with 100 nM [³H]LTB₄ in the absence or presence of 5 mM GSH. B, Membrane vesicles (50 µg of protein) from Sf9 cells expressing recombinant ABCC4 (Sf9-ABCC4) and control Sf9 cells (Sf9-Co) were incubated with 100 nM [³H]LTB₄ in the presence of 5 mM GSH. C, Membrane vesicles (30 µg of protein) from vector-transfected HeLa control (HeLa-Co) cells were incubated with 100 nM [³H]LTB₄ in the absence or presence of 5 mM GSH. Radioactivity and ATP-dependent transport were determined as described in the legend to Fig. 2. Data represent mean values ± S.D. from a triplicate determination reproduced independently at least once. **P*< 0.01 as compared with vesicles from control cells (B) and ***P*<0.001 as compared with the absence of GSH (A and C).

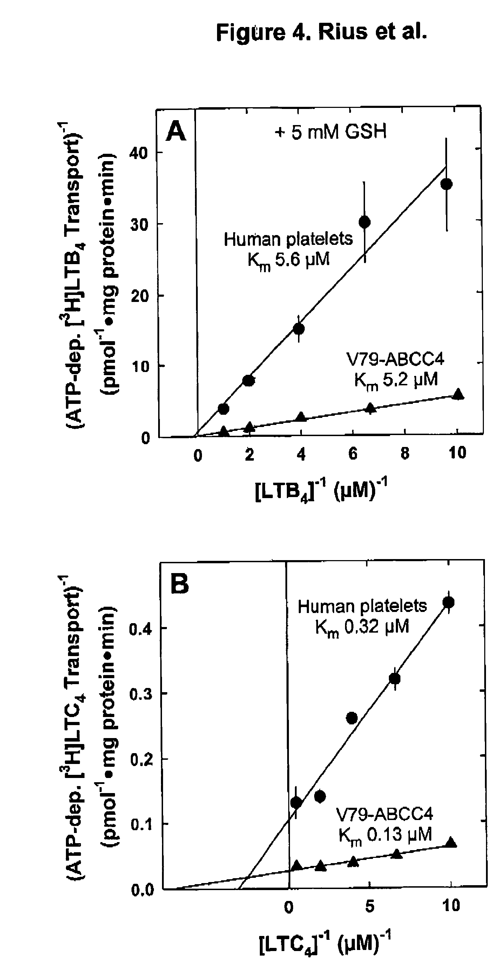

**Fig. 4****. Kinetic analysis of ABCC4-mediated [³H]LTB₄ and [³H]LTC₄ transport. Rates of** ATP-dependent transport of [³H]LTB₄ in the presence of 5 mM GSH (*A*) and of [³H]LTC₄ (B) were determined in membrane vesicles from *ABCC4*-transfected V79 cells (▲) and in plasma membrane fractions from human platelets (•) under substrate concentrations described in "Experimental Procedures. The Kₘ values were calculated from double-reciprocal plots. Data represent mean values ± S.D. from a triplicate determination.

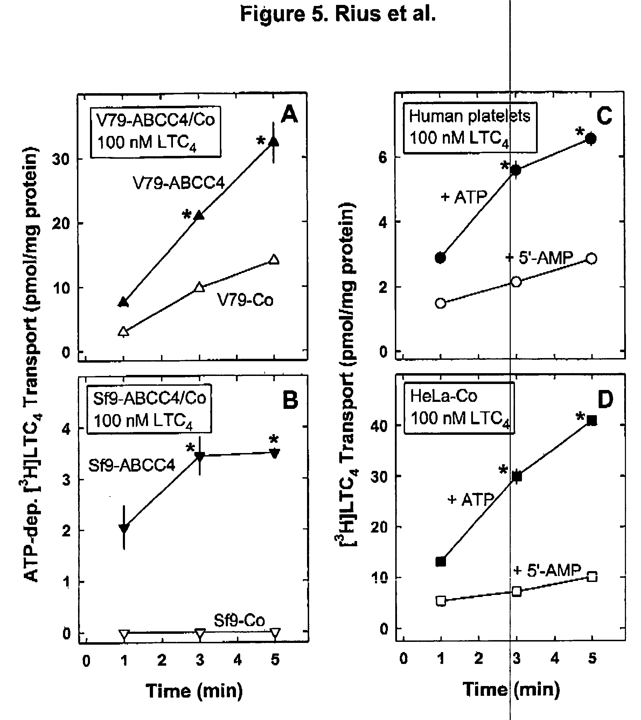

**Fig. 5****. Transport of LTC₄ into membrane vesicles from V79 and Sf9 cells containing recombinant ABCC4 protein (V79-ABCC4 and Sf9-ABCC4), from human platelets and from HeLa control cells. A** and *B*, Membrane vesicies (30-50 µg of protein) from ABCC4-expressing cells (V79-ABCC4 and Sf9-ABCC4) and control cells (V79-Co and Sf9-Co) were incubated with 100 nM [³H]LTC4. C and D, Membrane vesicles from human platelets (100 µg of protein) and HeLa control cells (HeLa-Co; 25 µg of protein) were incubated with 100 nM [³H]LTC₄. The vesicle-associated radioactivity was determined by filtration through nitrocellulose filters, The rates of ATP-dependent transport were calculated by subtracting transport in the presence of 4 mM 5'-AMP as a blank from transport in the presence of 4 mM ATP. Data represent mean values ± S.D. from a triplicate determination reproduced independently at least once. **P*<0.001 as compared with vesicles from control cells (*A* and *B*) or to the absence of ATP (*C* and *D*).

**Table 1. Inhibition of ABCC4-mediated LTB₄ transport in the presence of GSH**

| **Compounds** | **Concentration (µM)** | **ATP-dep. [³H]LTB₄ Transport (% of control)** |
|---|---|---|
| [³H]LTB₄ (control, 100 nM) | | 100 |
| 5-Hydroxy-6,8,11,14-eicosatetraenoaie | 1 | 86 ± 7 |
| 12-Hydroxy-5,8,10, 14-eicosatetraenoate | 1 | 106 ± 6 |
| LTC₄ | 1 | 39 ± 4 |
| LTD₄ | 1 | 67 ± 9 |
| S-Decyl GSH | 1 | 65 ± 6 |
| | 10 | 23 ± 3 |
| Sulindac sulfide | 1 | 38 ± 4 |
| | 10 | 26 ± 3 |
| Indomethacin | 10 | 32 ± 2 |
| Prostaglandin E₂ | 10 | 46 ± 5 |
| Cholyltaurine | 10 | 39 ± 2 |
| Dipyridamole | 10 | 57 ± 3 |
| MK571 | 10 | 60 ± 4 |
| | 20 | 47 ± 2 |
| Montelukast | 10 | 52 ± 5 |
| | 20 | 28 ± 2 |
| Probenecid | 100 | 73 ± 7 |
| Sulfinpyrazone | 100 | 73 ± 7 |

Membrane vesicles from V79-ABCC4 cells were incubated with 10C nM [³H]LTB₄ and 5 mM GSH for 5 min at 37 °C with several compounds. Rates of ATP-dependent [3H]TB₄ transport were determined as described and calculated as % of control. The rate of [³H]LTB₄ transport in the absence of inhibitors amounted to 0.75 ± 0.06 pmol x mg protein⁻¹ in 5 min. Data represent mean values ± SD from a triplicate determination.

**Table 2. Inhibition of ABCC4-mediated LTC₄ transport in platelets**

| **Compounds** | **Concentration (µM)** | **ATP-dep. [³H]LTC₄ Transport (% of control)** |
|---|---|---|
| [³H]LTC₄ (control, 100 nM) | | 100 |
| *S*-Decyl GSH | 1 | 42 ± 2 |
| | 10 | 11 ± 1 |
| Sulindac sulfide | 1 | 99 ± 5 |
| | 10 | 108 ± 2 |
| Sulindac sulfide + 5 mM GSH | 1 | 63 ± 6 |
| | 10 | 2 ± 0.5 |
| MK571 | 10 | 89 ± 6 |
| | 20 | 63 ± 3 |
| Montelukast | 10 | 75 ± 4 |
| | 20 | 57 ± 4 |

Plasma membrane vesicles from human platelets were incubated with 100 nM [³H]LTC₄ for 3 min at 37 °C in the presence of several compounds at the concentrations indicated. Except where indicated with suliridac sulfide, [³H]LTC₄ transport was measured in the absence of GSH. Rates of ATP-dependent [³H]LTC₄ transport were determined as described and calculated as % of control. The rate of [³H]LTC₄ transport in the absence of inhibitors amounted to 2.8 ± 0.2 pmol x mg protein⁻¹ in 3 min. Data represent mean values ± SD from a triplicate determination.

The invention is further explained by the following examples without being bound to it.

### Examples

### Examples 1: Experimental Procedures

***(a) Materials -*** [5,6,8,9,11,12,14,15-³H]Leukotriene B₄ (LTB₄; 165 Ci/mmol), [14,15,19,20-³H]leukotriene C₄ (LTC₄; 158 Ci/mmol), [14,15,19,20-³H]leukotriene D₄ (LTD₄; 167 Ci/mmol), and [³H]cholyltaurine (C-tau; 1.19 Ci/mmol) were purchased from Perkin Elmer (Boston, MA). LTC₄ was from Cayman Chemical (Ann Arbor, Ml) and MK571 was from Alexis Biochemicals (Lausen, Switzerland). Montelukast was purchased from Sequoia Research Products (Pangboume, United Kingdom). 5-Hydroxy-6,8,11,14-eicosatetraenoate was from Biomol International (Plymouth Meeting, PA) and Sephadex G-50 fine from Amersham Bioscience (Uppsala, Sweden). LTB₄, GSH, S-methyl GSH, S-decyl GSH, acivicin, dithiothreitol (DTT), 12-hydroxy-5,8,10,14-eicosatetrasnoate, prostaglandin E₂ (PGE₂), C-tau, dipyridamole, sulindac sulfide, indomethacin, probenecid, and sulfinpyrazone were obtained from Sigma (St. Louis, MO). Isolate membranes from Sf9 cells containing ABCC4 (Sf9-ABCC4) and Sf9 control cells (Sf9-Co) were purchased from Solvo Biotechnology (Budapest, Hungary).
***(b)*** ***Antibodies** -* The SNG antiserum was raised against the carboxy terminus of human ABCC4 (24). The protein affinity-purifed SNG antiserum was obtained as described (24). The monoclonal antibody QCRL-1 against ABCC1 was from Biozol (Eching, Germany), and horseradish peroxidase-conjugated goat anti-rabbit and anti-mou IgGs were from Bio-Rad (Munich, Germany).
***(c) Cell lines and cell culture -*** Chinese hamster lung V79 fibroblasts permanently expressing human recombinant ABCC4 (V79-ABCC4) were grown in Dulbecco's modified Eagle's medium (Sigma), supplemented with 10% (v/v) fetal bovine serum and 100 units/ml penicillin/streptomycin, and kept at 37°C and 5% CO₂, as described (24). Human embryonic kidney HEK293 cells were grown in minimum essential medium (Sigma), supplemented with 10% (v/v) fetal bovine serum and 100 units/ml penicillin/streptomycin, and kept at 37°C and 5% CO₂. HEK293 cells were transfected with the pREP9-*ABCC*4 cDNA construct or the vector only (24), using FuGENE 6 transfection reagent (Roche Diagnostics, Mannheim, Germany). After 48 h, the cells were split, and stable transfectants were selected using medium with 1 mg/ml G418 (Gibco, Karlsruhe, Germany). Resistant clones were screened by immunoblot analysis and immunofluorescence microscopy for ABCC4 expression as described (24). Sodium butyrate (5 mM) was added to the cells 24 h before harvesting to enhance the expression of the recombinant protein (37). The non-P-glycoprofiein-expressing doxorubicin-resistant HL60-ADR cells (38,39) were grown in RPMI medium (Sigma), supplemented with 10% (vlv) fetal bovine serum, 100 units/ml penicillin/streptomycin, and 200 nM daunorubicin, and kept at 37°C and 5% CO₂ (14). The vector control transfected HeLa cells were grown in RPMI medium, supplemented with 10% (v/v) fetal bovine serum and 100 units/ml penicillin/streptomycin (40).
***(d) Preparation of membrane vesicles from human platelets -* P**lasma membrane fractions from human platelets were isolated as described (33,41,42). Briefly, platelet-rich plasma was obtained from the Blood Center of the University of Heidelberg hospitals. The platelets were disrupted in homogenization buffer (100 mM KCl, 25 mM NaCl, 2 mM MgSO₄, 12 mM Na₃ citrate, 10 mM D-glucose, 25 mM Hepes, 5 mM ATP, 0.35% BSA, pH 7.0) by freezing in liquid nitrogen and thawing at 37 °C four times. The homogenate was layered onto a linear 30-60 % sucrose density gradient. After ultracentrifugation at 200,000 x g for 60 min at 4 °C, four fractions were collected, homogenized, and washed by centrifugation at 100,000 x g in incubation buffer (250 mM sucrose, 10 mM Tris/HCl, pH 7.4), and passed 20 times through a 27-gauge needle for vesicle formation. Aliquots of the membrane vesicle suspension were stored in liquid nitrogen.
***(e) Preparation* of *membrane vesicles from human erythrocytes*** - Inside-out plasma membrane vesicles from human erythrocytes were prepared as described (43,44). Briefly, human blood was obtained from a normal healthy donor and collected into EDTA syringes. Erythrocytes were washed three times by centrifugation at 2,300 x g for 10 min at 4 °C, discarding the buffy coat each time from the surface of the pellet. Hemolysis was achieved in 5P8 buffer (5 mM sodium phosphate, pH 8.0), followed by centrifugation at 25,000 x g for 10 min at 4 °C. The resulting membranous ghosts were suspended in 0.5P8 buffer (1:10 dilution of 5P8 buffer) and slowly mixed for 3 h on ice. After centrifugation at 25,000 x g (10 min at 4 °C), the membranes were washed by centrifugation at 40,000 x g (30 min at 4 °C) in 0.5P8 buffer. The membranes were resuspended in incubation buffer (250 mM sucrose, 10 mM Tris/HCl, pH7.4) and passed 10 times through a 27-gauge needle for vesicle formation. Aliquots of the membrane vesicle suspension were frozen and stored in liquid nitrogen.
***(f) Preparation of membrane vesicles from mammalian cell lines -*** Inside-out plasma membrane vesicles from transfected V79 and HEK233 cells, HL60-ADR, and control HeLa cells were prepared as described (45). Briefly, the cells were lysed by incubation in hypotonic buffer (0.1 mM EDTA, 0.5 mM sodium phosphate, pH 7.0) for 1.5 h, followed by homogenization with a Potter-Etvehjem homogenizer. After centrifugation of the homogenate at 12,000 x g (10 min at 4 °C), the postnuclear supernatant was centrifuged at 100,000 x g for 45 min at 4 °C. The resulting pellet was suspended in incubation buffer (250 mM sucrose, 10 mM Tris/HCl, pH 7.4), homogenized with a tight-fitting Dounce (glass/glass) homogenizer, and layered over 38 % sucrose in 5 mM HEPES/KOH, pH 7.4. After centrifugation at 280,000 x g for 1.5 h at 4°C, the turbid layer at the interface was collected, washed by centrifugation in the incubation buffer (100,000 x g), and passed 20 times through a 27-gauge needle for vesicle formation. Aliquots of the membrane vesicle suspension were frozen and stored in liquid nitrogen.
***(g) Immunoblot analysis*** - Membrane vesicles (10-50 µg protein) from cell lines and human platelets and erythrocytes were diluted with sample buffer and incubated at 37 °C for 30 min before their separation on a 7.5 % SDS polyacrylamide gel. Immunoblotting was performed using a tank blotting system (Bio-Rad) and an enhanced chemiluminescence horseradish peroxidase detection system (Amersham Biosciences). The polyclonal SNG (24) antiserum was diluted 1:1000 in PBS containing 0.05 % Tween 20, and the protein affinity-purified SNG antiserum and the monoclonal QCRL-1 antibody were diluted 1:500 (46). For peptide competition studies, the SNG antiserum (1:1000 dilution) or the affinity-purified SNG antiserum (1 :500 dilution) was incubated for 16 h at 4 °C with 10 µM of the synthetic peptide used to generate the SNG antiserum, and then applied onto the blots as the primary antibody. The horseradish peroxidase-conjugated goat anti-rabbit and anti-mouse antibodies were used at dilutions of :2000 and 1:3000, respectively.
*(h) **Vesicle transport studies** -* ATP-dependent transport of [³H]LTB₄ into inside-out membrane vesicles was measured by centrifugation through Sephadex G-50 columns essentially as described (45). This procedure is more efficient for the separation of extravesicular labeled substrates than the filtration through nitrocellulose filters, as labeled lipophilic substrates bound to the filters may cause high background radioactivity. Membrane vesicles were incubated at 37 °C with 4 mM ATP, 10 mM MgCl₂, 10 mM creatine phosphate, 100 µg/ml creatine kinase, labeled substrate, the presence or absence of 5 mM GSH, in incubation buffer containing 250 mM sucrose, and 10 mM Tris/HCl at pH 7.4. DTT (1 mM) was added to the incubation buffer for measurements in the presence and absence of GSH. The final incubation volume was 55 µl. The substrate and inhibitor concentrations are given in the respective figure legends and tables. For inhibition studies, compounds were added from a stock solution in an appropriate solvent (incubation buffer, ethanol, or dimethyl sulfoxide at a final concentration of the solvent below 0.5 % v/v) and identical concentrations of the solvent were used in control samples. NICK spin columns (0.2 g of Sephadex G-50 per 3.3 ml of incubation buffer) were prepared by rinsing with incubation buffer and were centrifuged at 400 x g for 3 min at 4 °C immediately before use. Aliquots (15 or 20 µl) of the incubations were taken at the times indicated, diluted with ice-cold incubation buffer (final volume 100 µl), and immediately loaded onto the Sephadex G-50 columns. The columns were additionally rinsed with 100 µl of incubation buffer and centrifuged at 400 x g for 3 min at 4 °C. The effluents were collected, dissolved in liquid scintillation fluid, and counted for radioactivity. In control experiments, ATP was replaced by an equal concentration of AMP-PCP. Rates of net ATP-dependent transport were calculated by subtracting values obtained in the presence of AMP-PCP as a blank from those in the presence of ATP.

ATP-dependent transport of [³H]LTC₄ and [³H]C-tau into inside-out membrane vesicles was measured by rapid filtration through nitrocellulose filters as described (45). Transport of [³H]LTC₄ and [³H]C-tau were performed essentially as described above except that 5'-AMP instead of ATP was used as a blank and that aliquots of the incubations were filtered immediately through nitrocellulose filters (0.2-µm pore size, Millipore, Billerica, MA) pre-soaked in incubation buffer. Filters were rinsed with 8 ml of incubation buffer, dissolved in liquid scintillation fluid, and counted for radioactivity.

For determination of kinetic constants, transport rates were measured at five different substrate concentrations (0.1-1 µM for LTB₄, 1-10 mM for GSH, and 0.1-2 µM for LTC₄). Kₘ values were determined as the substrate concentration at half-maximal velocity of transport under these conditions by use of double-reciprocal plots and direct curve-fitting to the Michaelis-Menten equation.

For the statistical analysis, Student's *t*-test was used. A value < 0.01 was considered significant, and a *P* value < 0.001 was considered highly significant.

### EXAMPLE 2: RESULTS

***(a) Cellularsystems to study endogenous and recombinant ABCC4* -** Several cellular preparations were examined for the endogenous expression of ABCC4 as possible tools for transport studies. Consistent with previous findings, the polyclonal SNG antiserum detected broad bands between 170 and 200 kDa in membrane vesicles from human platelets and erythrocytes (Fig. 1A, upper panel), as well as in tissue homogenates from prostate (28,29,33,34). In erythrocytes, we detected an additional band at 120 kDa, which was probably the result of protein degradation. Strong signals were also detected in membrane vesicles from HL60-ADR and HeLa-Co cells, indicating the endogenous expression of ABCC4 in these ceii lines (Fig. 1A, upper panel). A similar pattern of bands was obtained with the affinity-purified SNG antiserum (data not shown). The ABCC4-specific signal was abolished in all samples when the SNG or the affinity-purified SNG antisera were incubated with the synthetic SNG peptide before immunoblot analysis (data not shown).
   Because of the high levels of endogenous ABCC4 in the cell and tissue preparations (Fig. 1*A*, upper panel), ABCC1 levels were analyzed under the same conditions to exclude cross-reactivity of the SNG antiserum (Fig. 1*A*, lower panel). The monoclonal QCRL-1 antibody detected a broad band between 170 and 190 kDa in vesicles from HL60-ADR cells, as well as a faint band at 200 kDa in vesicles from HeLa-Co cells (Fig. 1*A*, lower panel). However, with the identical protein amount of 10 µg and equal exposure time, no ABCC1 was detected in the plasma membrane vesicles from human platelets and erythrocytes or in tissue homogenates from prostate (Fig. 1*A*, lower panel). ABCC1 protein in human erythrocytes was detectable only after longer exposure times or with greater protein amounts (data not shown).
   Increasing amounts of membrane vesicles from human platelets were examined to compare the detection of ABCC4 and ABCC1 (Fig. 1*B*). The SNG antiserum detected greater signal intensity as the amount of protein increased, whereas the QCRL-1 antibody detected no protein at all (Fig. 1*B*). A weak signal for ABCC1 was obtained only when the exposure time of the blot was extended 20 times as long as in Fig. 1*B* (data not shown). These results indicate that human platelets and HeLa-Co cells are a rich source of endogenous ABCC4 and have very low levels of endogenous ABCC1 (Fig. 1*A* and *B*).
   Cell lines expressing recombinant ABCC4 were also examined by immunoblotting (Fig. 1C). The SNG antiserum detected a broad band at 170 to 190 kDa in membrane vesicles from hamster V79 fibroblasts and HEK293 cells transfected with recombinant *ABCC4* (Fig. 1C). The vesicles from the HEK293 cells transfected with the empty vector also showed a significant level of endogenous ABCC4 protein (Fig. 1C). Vesicles from the Sf9 insect cells containing recombinant human *ABCC4* presented a narrow band at 150 kDa, consistent with expression in a cell system which lacks complex glycosylation (30,47).
***(b) ATP-dependent transport by ABCC4 of the standard substrate C-tau in the presence* of** ***GSH** -* Membrane vesicles containing endogenous or recombinant ABCC4 were assayed for transport activity using 5 µM [³H]C-tau in the presence or absence of 5 mM GSH during a 10-min period. Among the four membrane fractions isolated from human platelets, the fraction containing mainly plasma membranes showed the highest stimulation of the ATP-dependent [³H]C-tau transport by GSH or S-methyl GSH. Membrane vesicles from the platelet plasma membrane fraction showed an increase in ATP-dependent [³H]C-tau transport from 0.7 to 2.8 pmol x mg protein⁻¹ x min⁻¹ in the presence of GSH. Therefore, further transport studies were performed using the membrane vesicles from the plasma membrane fraction of human platelets. In membrane vesicles from HeLa-Co cells, ATP-dependent [³H]C-tau transport was greatly increased by GSH (without GSH: 1.7; with GSH 14.3 pmol x mg protein⁻¹ x min⁻¹).
   Recombinant ABCC4 was also assayed for transport of C-tau in the presence of GSH. Membrane vesicles from ABCC4 containing V79 cells, HEK-ABCC4, and Sf9-ABCC4 cells were highly active in GSH-dependent C-tau transport (data not shown).
***(c) GSH increased ATP-dependent transport of LTB₄ into membrane vesicles from V79, HEK293, and Sf9 cells containing recombinant ABCC4* -** ATP-dependent transport of [³H]LTB₄ at a concentration of 100 nM was measured into membrane vesicles from the *ABCC4-* and vector-transfected V79 cells (Fig. 2A and B). The rates of ABCC4-mediated [³H]LTB₄ transport increased 4.7-fold in the presence of 5 mM GSH (Fig. 2A). A low rate of LTB₄ transport was detected in membrane vesicles from vector-transfected V79 cells (Fig. 2A), which is in line with the presence of hamster Abcc4 in the V79 cells (24,25). Similar results were obtained with the non-reducing GSH analog S-methyl GSH (data not shown).
   Using membrane vesicles from the ABCC4- and vector-transfected HEK293 cells, the effects of GSH on the ATP-dependent transport of [³H]LTB₄ were similar to those with the V79 cells (Fig. 2C and D). In the absence of GSH, LTB₄ transport was negligible (Fig. 2D); however, addition of GSH caused an increase of the LTB₄ transport into membrane vesicles from both HEK-ABCC4 and HEK-Co cells (Fig. 2C). The transport rates in the presence of GSH were proportional to the corresponding levels of recombinant and endogenous ABCC4 protein in HEK293 cells (Fig. 1 C). Membrane vesicles from Sf9 cells expressing recombinant ABCC4 confirmed the ABCC4-mediated LTB₄ transport in the presence of GSH (Fig. 3*B*).
   Thus, with three different cellular sources of recombinant ABCC4, ABCC4 was recognized as an ATP-dependent transporter of LTB₄ in the presence but not in the absence of GSH or S-methyl GSH (Fig. 2 and Fig. 3*B*).
**(*d*) *GSH increased ATP-dependent transport of LTB₄ into membrane vesicles from human platelets and HeLa control* cells *containing endogenous ABCC4 -*** The ABCC4-mediated transport of [³H]LTB₄ was further examined by transport studies with membrane preparations containing endogenous ABCC4. Using vesicles from plasma membranes of human platelets, we observed a significant ATP-dependent transport of [³H]LTB₄ only when GSH was present (Fig. 3A). To verify whether the low level of endogenous ABCC1 and ABCC5 could play a role in the LTB₄ transport in membrane vesicles from platelets, [³H]LTB₄ transport was measured in vesicles from *ABCC5-*containing V79 cells (48) and from ABCC1-containing HeLa cells (15). Neither ABCC5 nor ABCC1 mediated [³H]LTB₄ accumulation in vesicles in the presence or absence of GSH (data not shown). However, vesicles from HeLa control cells showed a pronounced and time-dependent ATP-dependent LTB₄ transport in the presence of GSH (Fig. 3C). These results indicate that LTB₄ transport by vesicles from human platelets and HeLa cells is mediated by endogenous ABCC4 in the presence of GSH.
***(e) Kinetic analysis of ABCC4-mediated ATP-dependent LTB₄ transport in the presence* of *GSH -*** To determine the affinity of ABCC4 for LTB₄, we used vesicles containing recombinant ABCC4 from V79 cells and vesicles containing endogenous ABCC4 from human platelets (Fig. 4A). We tested the affinities in a concentration range from 0.1 to 1 µM LTB₄ in the presence of 5 mM GSH. Kₘ values for ABCC4 were 5.2 ± 0.2 µM in vesicles from V79-ABCC4 and 5.6 ± 0.6 µM in vesicles from human platelets (Fig. 4A). The effect of GSH concentrations between 1 and 10 mM on the ATP-dependent [³H]LTB₄ transport was studied in vesicles from V79-ABCC4 cells. At a constant concentration of 100 nM LTB₄, the Kₘ value of GSH for ABCC4 was 1.1 ± 0.2 mM (not shown).
***(f)* Inhibition of *the ABCC4-mediated LTB₄* transport in *the presence* of GSH -** Several organic anions were tested for inhibition of the ATP-dependen [³H]LTB₄ transport in the presence of GSH into vesicles from V79-ABCC4 cells (Table 1). At a concentration of 1 µM, 5- and 12-hydroxyeicosatetraenoate did not cause a significant inhibition in the presence of GSH. In contrast, 1 µM LTC₄ was a potent inhibitor of the transport, causing 61 % inhibition, 1 µM LTD₄ caused 33 % inhibition, and the GSH derivative and LTC₄ analog, *S*-decyl glutathione at 10 µM caused 74 % inhibition. The non-steroidal anti-inflammatory drugs sulindac sulphide and indomethacin caused 74 % and 68 % inhibition, respectively, at 10 µM (Table 1). In the presence of GSH, the LTD₄ receptor antagonists MK571 and montelukast were also effective inhibitors of the LTB₄ transport. MK571 at 20 µM caused an inhibition of LTB₄ transport in the presence of GSH with a Ki value of 9.8 µM. In addition, several known ABCC4 substrates and ABCC inhibitors produced a significant inhibition (Table 1).
***(g) ATP-dependent transport of LTC₄ into membrane vesicles from V79, HEK293, and Sf9 cells and from human platelets and HeLa cells-*** Because LTC₄ potently inhibited LTB₄ transport in the presence of GSH, we measured transport of [³H]LTC₄ in vesicles containing recombinant ABCC4 from V79, HEK293, and Sf9 cells during a 5-min period (Fig. 5A and *B*). Surprisingly, ATP-dependent LTC₄ accumulation at a concentration of 100 nM amounted to 32.3 ± 3.2 pmol x mg protein⁻¹ after 5 min into vesicles from ABCC4-transfected V79 cells and to 14 ± 0.5 pmol mg protein⁻¹ into vesicles from vector-transfected V79 cells (Fig. 5A). The accumulation of LTC₄ resulted in a 2.2-fold higher transport rates in vesicles from V79-ABCC4 cells th an in vesicles from V79-Co cells. This ABCC4-mediated transport was confirmed in vesicles containing recombinant ABCC4 from HEK293 cells and from Sf9 cells (Fig. 5*B*). To further characterize the LTC₄ transport in membranes containing endogenous ABCC4, vesicles from human platelets were used (Fig. 5C). These vesicles, which contained very low levels of ABCC1 (Fig. 1A and *B*), mediated a significant transport of LTC₄ with a transport rate of 0.6 pmol x mg protein⁻¹ x min⁻¹ (Fig. 5C). Membrane vesicles from HeLa cells, which also showed low levels of endogenous ABCC1 (Fig. 1A), supported LTC₄ transport at a high rate (Fig. 5D). Addition of GSH or S-methyl GSH did not stimulate the transport of LTC₄ in vesicles containing recombinant or endogenous ABCC4 (data not shown). ATP-dependent transport of [³H]LTD₄ was also observed in vesicles from V79-ABCC4 cells with a rate of 0.6 pmol x mg protein⁻¹ x min⁻¹ in the absence of GSH.
   The affinity of ABCC4 for LTC₄ was determined in vesicles containing recombinant ABCC4 from V79 cells (Fig. 4*B*), vesicles containing endogenous ABCC4 from platelets (Fig. 4B), and vesicles from V79 control cells. In a concentration range from 0.1 to 2 µM LTC₄ we determined Kₘ values for ABCC4 of 0.13 ± 0.02 µM in vesicles from V79-ABCC4 and 0.32 ± 0.03 µM in vesicles from human platelets (Fig. 4A). The Vₘₐₓ values were 37.4 pmol x mg protein⁻¹ x min⁻¹ for V79-ABCC4 and 9.7 pmol x mg protein⁻¹ x min⁻¹ for human platelets.
   In vesicles from human platelets, LTC₄ transport was measured in the presence of several organic anions, especially LTD₄ receptor antagonists (Table 2). Montelukast and MK571 at a concentration of 20 µM caused 43 and 32 % inhibition, respectively. In addition, S-decyl glutathione caused 58 % inhibition at a concentrati on of 1 µM. Sulindac sulfide, in the absence of GSH and at a concentration of 10 µM, did not inhibit the LTC₄ transport in platelets. However, sulindac sulfide caused 90 % inhibition in the presence of 5 mM GSH (Table 2).

### EXAMPLE 3: DISCUSSION

LTB₄ is an important mediator of the inflammatory process. In the present invention, we have identified ABCC4 as a novel and so far as the only efflux pump for LTB₄. Our conclusions are based on experiments in several membrane vesicle preparations that contain recombinant or endogenous human ABCC4 (Figs. 1-3). Moreover, millimolar concentrations of GSH were an absolute requirement for the ATP-dependent transport of LTB₄ (Figs. 2 and 3), analogous to the ATP-dependent transport of most bile acids by ABCC4 (24,25). In this study, we did not determine whether GSH is co-effluxed with LTB₄, as is the case with cholyltaurine (24,25) or whether binding of GSH or S-methyl GSH to ABCC4 is sufficient. The LTB₄ efflux via ABCC4 in the presence of GSH is physiologically relevant, since millimolar concentrations of GSH occur in living cells (26).

Our finding that LTC₄ is a full substrate for ABCC4 in the absence of GSH is consistent with the structural differences between the molecules: LTB₄ lacks the covalently bound GSH moiety of LTC₄. The high-affinity transport of LTC₄ by ABCC4 (Figs. 4 and 5) contributes to an explanation of the active efflux transport of LTC₄ observed with blood platelets (11) and erythrocyte membrane vesicles (49). Both platelets and erythrocytes contain much more ABCC4 than ABCC1 (Fig. 1*A* and *B*). The abundance of ABCC4 observed in the plasma membrane of these cells confirms recent studies from other laboratories (33,34). Therefore, most ATP-dependent LTC₄ efflux from platelets and erythrocytes is likely mediated by ABCC4.

Efflux of LTC₄ from platelets and of LTB₄ from erythrocytes and endothelial cells represents the final step in the transcellular synthesis of these LTs from their precursor LTA₄ (3,10,11,50). ABCC4 may thus play an important role in the transcellular synthesis of LTB₄ and LTC₄. ABCC4 may be termed an eicosanoid efflux pump n view of its broad substrate spectrum that includes, in addition to LTB₄ and LTC₄, prostaglandin (PG) E₂ (51), PGF₂ₐ, and thromboxane B₂ (29).

The present invention adds several inhibitors of ABCC4-mediated transport to those identified in recent years (24,30,33,51,52). In this study, potent inhibitory compounds of ATP-dependent transport of LTB₄ in the presence of 5 mM GSH included LTC₄ and its structural analog S-decyl GSH, the anti-inflammatory agent sulindac sulfide, and the LTD₄ receptor antagonists montelukast and MK571 (Table 1). The latter substances also interfered with the ABCC4-mediated transport of LTC₄ in the absence of GSH (Table 2). Some of these compounds were identified formerly as inhibitors of ABCC1, as described for MK571 (14,15,45) and S-docyl GSH (22). Thus, the inhibitory selectivity of these structural analogs of LTD₄ and LTC₄ may not be sufficient to discriminate between ABCC1 and ABCC4. This observation is surprising since both ABC transporters share only 39 % amino acid sequence identity and differ largely by the absence of the amino-terminal extension in ABCC4 (21). Several of the inhibitors of LTB₄ transport by ABCC4 (Table 1) are established anti-inflammatory agents, such as indomethacin and sulindac sulfide. Inhibition of ABCC4 and thereby interference of the transcellular synthesis of LTB₄ and LTC₄ may contribute to the anti-inflammatory action of some anti-inflammatory drugs. However, anti-inflammatory agents must first be taken up by a target cell before they can interact with ABCC4 on its cytosolic domain, which was exposed in our inhibition studies with inside-out membrane vesicles. Moreover, the distribution and substrate specificity of the cellular uptake transporters and the site of drug administration in the body may determine whether an inhibitor of ABCC4 exerts anti-inflammatory actions.

Over the past years, ABCC4 has been recognized as a transporter of broad substrate specificity, including endogenous substrates, drugs, and drug candidates (17,24,25,28-31,33,47,51,52). The physiological function of human ABCC4 cannot be defined by the in vitro substrate specificity alone. Rather, that role may depend on the cell type and the level of its expression, in addition to the presence or absence of other ABCC subfamily members, such as ABCC1, ABCC3, or ABCC5. Our present work was focused on two novel substrates of ABCC4 and indicated that this eicosanoid transporter may play an important role in the biosynthetic release and in transcellular biosynthesis of LTB₄ and LTC₄. Studies on more potent and selective inhibitors of ABCC4 may be an attractive aim in drug development.

### EXAMPLE 4: REFERENCES

1. Samuelsson, B. (1983) Science 220, 568-575
2. Funk, C. D. (2001) Science 294, 1871-1875
3. Folco, G. and Murphy, R. C. (2006) Pharmacol.Rev. 58, 375-388
4. Lam, B. K., Owen, W. F., Jr., Austen, K F., and Soberman, R. J. (1989) J. Biol. Chem. 264, 12885-12889
5. Lam, B. K., Gagnon, L., Austen, K. F., and Soberman, R. J. (1990) J.Biol.Chem. 265, 13438-13441
6. Lam, B. K., Xu, K., Atkins, M. B., and Austen, K. F. (1992) Proc.Natl.Acad.Sci.U.S.A 89, 11598-11602
7. Schaub, T., Ishikawa, T., and Keppler, D. (1991) FEBS Lett. 279, 83-86
8. Ishikawa, T., Müller, M., Klünemann, C., Schaub, T., and Keppler, D. (1990) J.Biol.Chem. 265, 19279-19286
9. Keppler, D. (1992) Rev.Physiol Biochem.Pharmacol. 121, 1-30
10. Maclouf, J. A. and Murphy, R. C. (1988) J.Biol.Chem. 263, 174-181
11. Sjölinder, M., Tornhamre, S., Claesson, H. E., Hydman, J., and Lindgren, J. (1999) J.Lipid Res. 40, 439-446
12. Fitzpatrick, F. A., Liggett, W. F., and Wynalda, M. A. (1984) J.Biol.Chem. 259, 2722-2727
13. Cole, S. P., Bhardwaj, G., Gerlach, J. H., Mackie, J. E., Grant, C. E., Almquist, K. C., Stewart, A. J., Kurz, E. U., Duncan, A. M., and Deeley, R. G. (1992) Science 258, 1650-1654
14. Jedlitschky, G., Leier, I., Buchholz, U., Center, M., and Keppler, D. (1994) Cancer Res. 54, 4833-4836
15. Leier, I., Jedlitschky, G., Buchholz, U., Cole, S. P., Deeley, R. G., and Keppler, D. (1994) J.Biol.Chem. 269,27807-27810
16. Leier, I., Jedlitschky, G., Buchholz, U., and Keppler, D. (1994) Eur.J.Biochem. 220, 599-606
17. Kruh, G. D. and Belinsky, M. G. (2003) Oncogene 22, 7537-7552
18. König, J., Nies, A. T., Cui, Y., Leier, I., and Keppler, D. (1999) Biochim.Biophys.Acta 1469, 377-394
19. Nies, A. T. and Keppler, D. (2007) Pflügers Arch. 453, 643-659
20. Borst, P., Evers, R., Kool, M., and Wijnholds, J. (2000) J.Natl.Cancer Inst. 92, 1295-1302
21. Deeley, R. G., Westlake, C., and Cole, S. P. (2006) Physiol Rev. 86, 849-899
22. Loe, D. W., Almquist, K. C., Deeley, R. G., and Cole, S. P. (1996) J.Biol.Chem. 271, 9675-9682
23. Cole, S. P. and Deeley, R. G. (2006) Trends Pharmacol. Sci. 27, 438-446
24. Rius, M., Nies, A. T., Hummel-Eisenbeiss, J., Jedlitschky, G., an Keppler, D. (2003) Hepatology 38, 374-384
25. Rius, M., Hummel-Eisenbeiss, J., Hofmann, A. F., and Keppler, D, (2006) Am. J. Physiol Gastrointest. Liver Physiol 290, G640-G649
26. Meister, A. (1988) J.Biol.Chem. 263, 17205-17208
27. Belinsky, M. G., Bain, L. J., Balsara, B. B., Testa, J. R., and Kruh G. D. (1998) J.Natl. Cancer Inst. 90, 1735-1741
28. Lee, K., Klein-Szanto, A. J., and Kruh, G. D. (2000) J.Natl. Cance Inst. 92, 1934-1940
29. Rius, M., Thon, W. F., Keppler, D., and Nies, A. T. (2005) J.Urol. 174, 2409-2414
30. van Aubel, R. A., Smeets, P. H., Peters, J. G., Bindels, R. J., and Russel, F. G. (2002) J.Am.Soc.Nephrol, 13, 595-603
31. Leggas, M., Adachi, M., Scheffer, G. L., Sun, D., Wielinga, P., Du, G., Mercer, K. E., Zhuang, Y., Panetta, J. C., Johnston, B., Scheper, R. J., Stewart. C. F., and Schuetz, J. D. (2004) Mol. Cell Biol. 24, 7612-7621
32. Nies, A. T., Jedlitschky, G., König, J., Herold-Mende, C., Steiner, H. H., Schmitt, H. P., and Keppler, D. (2004) Neuroscience 129, 349-360
33. Jedlitschky, G., Tirschmann, K., Lubenow, L. E., Nieuwenhuis, H. K., Akkerman, J. W., Greinacher, A., and Kroemer, H. K. (2004) Blood 104, 3603-3610
34. Klokouzas, A., Wu, C. P., van Veen, H. W., Barrand, M. A., and ladky, S. B. (2003) Eur.J.Biochem. 270, 3696-3708
35. Kool, M., de Haas, M., Scheffer, G. L., Scheper, R. J., van Eijk, M. J., Juijn, J. A., Baas, F., and Borst, P. (1897) Cancer Res, 57, 3537-3547
36. Szakacs, G., Annereau, J. P., Lababidi, S., Shankavaram, U., Arciello, A., Bussey, K. J., Reinhold, W., Guo, Y., Kruh, G. D., Reimers, M., Weinstein, J. N., and Goftesman, M. M. (2004) Cancer Cell 6, 129-137
37. Cui, Y., König, J., Buchholz, J.K., Spring, H., Leier, I., and Keppler, D. (1999) Mol.Pharmacol. 55, 929-937
38. Marquardt, D., McCrone, S., and Center, M. S. (1990) Cancer Res. 50, 1426-1430
39. Krishnamachary, N. and Center M. S. (1993) Cancer Res. 53, 3658-3661
40. Grant, C. E., Valdimarsson, G., Hipfner, D. R., Almquist, K, C., Cole, S. P., and Deeley, R. G. (1994) Cancer Res. 54,357-361
41. Rendu, F., Lebret, M., Nurden, A. T., and Caen, J. P. (1982) Br.J. Haematol. 52, 241-251
42. Broekman, M. J. (1992) Methods Enzymol. 215, 21-32
43. Steck, T. L., Weinstein, R. S., Straus, J. H., and Wallach, D. F. (1970) Science 168, 255-257
44. Steck, T. L. and Kant, J. A. (1974) Methods Enzymol. 31, 172-180
45. Keppler, D., Jedlitschky, G., and Leier, I. (1998) Methods Enzymol. 292, 607-616
46. Hipfner, D. R., Gauldie, S. D., Deeley, R. G., and Cole, S. P. (1994) Cancer Res. 54, 5788-5792
47. Chen, Z. S., Lee, K., and Kruh, G. D. (2001) J. Biol. Chem. 276, 33747-33754
48. Jedlitschky, G., Burchell, B., and Keppler, D. (2000) J.Biol.Chem. 275, 30069-30074
49. Pulaski, L., Jedlitschky, G., Leier, I., Buchholz, U., and Keppler, D. (1996) Eur.J.Biochem. 241, 644-648
50. Haeggström, J. Z. (2000) Am.J.Respir.Crit Care Med. 161, S25-S31
51. Reid, G., Wielinga, P., Zelcer, N., van der Heijden, I., Kuil, A., de Haas, M., Wijnholds, J., and Borst, P. (2003) Proc.Natl.Acad.Sci.U.S.A 100 9244-9249
52. Chen, Z. S., Lee, K., Walther, S., Raftogianis, R. B., Kuwano, M., Zeng, H., and Kruh, G. D. (2002) Cancer Res. 62, 3144-3150

## Claims

1. A method of treating a patient having an inflammatory disease, the method comprising using a therapeutically effective amount of a compound which inhibits the ABCC4-mediated release of leukotrienes.

2. The method according to claim 1, wherein the leukotriene is LTC or LTB4

3. The method according to claim 2, wherein the leukotriene is LTB4.

4. The method according to at least one of the claims 1-3, wherein the inhibitor is selected from the group consisting of S-Decyl GSH, sulindac sulfide, indomethacin, prostaglandin E₂, cholyltaurine, dipyridamole, MK571, montelukast, probenecid, and sulfinpyrazone.

5. The method according to any of claims 1 to 4, wherein the inflammatory disease is a disease of the respiratory tract.

6. A method of screening for a compound which inhibits the ABCC4-mediated release of leukotrienes, the method comprising
(a) contacting cells expressing ABCC4 with the compound under investigation and / or contacting membrane vesicles containing ABCC4 with the compound under investigation, and
(b) measuring the amount of released leukotrienes,
wherein a lower amount of released leukotrienes in the cells / membrane vesicles of (a) as compared to cells or membrane vesicles that are not contacted with said compound is indicative of an inhibitory effect of said compound.

7. The method according to claim 6, wherein the leukotrienes are radioactively labelled.

8. Use of a compound which inhibits the ABCC4-mediated release of leukotrienes for the preparation of a medicament for treating a patient having an inflammatory disease.

9. The use according to claim 8, wherein the leukotriene is LTC4 or LTB4.

10. The use according to Claim 9, wherein the leukotriene is LTB4.

11. The use according to any of claims 8 to 10, wherein the inhibitor is selected from the group consisting of S-Decyl GSH, sulindac sulfide, indomethacin, prostaglandin E₂, cholyltaurine, dipyridamole, MK571, montelukast, probenecid, and sulfinpyrazone.

12. The use according to any of claims 8 to 11, wherein the the inflammatory disease is a disease of the respiratory tract.

13. A method for manufacturing a medicament containing a therapeutically effective amount of a compound which inhibits the ABCC4-mediated release of leukotrienes.

14. A medicament containing a therapeutically effective amount of a compound which inhibits the ABCC4-mediated release of leukotrienes.

15. The medicament of claim 14, wherein the compound is a sulindac derivative or montelukast.
